**Europäisches Patentamt**

**European Patent Office**

**Office européen des brevets**

(19)

(11) Veröffentlichungsnummer : **0 617 289 A2**

## (12) EUROPÄISCHE PATENTANMELDUNG

(21) Anmeldenummer : **94890054.3**

(22) Anmeldetag : **09.03.94**

(51) Int. Cl.$^5$ : **G01N 33/96, // G01N33/92**

(30) Priorität : **19.03.93 AT 553/93**

(43) Veröffentlichungstag der Anmeldung :
**28.09.94 Patentblatt 94/39**

(84) Benannte Vertragsstaaten :
**AT BE CH DE DK ES FR GB IE IT LI NL PT SE**

(71) Anmelder : **IMMUNO Aktiengesellschaft**
**Industriestrasse 67**
**A-1221 Wien (AT)**

(72) Erfinder : **Lang, Hartmut, Dr.**
**Färbermühlgasse 13**
**A-1230 Wien (AT)**
Erfinder : **Molinari, Ewald, Dr.**
**Brühlerstrasse 79/3/14**
**A-2340 Mödling (AT)**
Erfinder : **Pichler, Peter, Dipl.-Ing.**
**Schmidgasse 13/10**
**A-1080 Wien (AT)**

(74) Vertreter : **Weinzinger, Arnulf, Dipl.-Ing. et al**
**Patentanwälte**
**Sonn, Pawloy, Weinzinger & Wolfram**
**Riemergasse 14**
**A-1010 Wien (AT)**

(54) **Lyophilisiertes Kontroll- oder Referenzplasma bzw. Kontroll- oder Referenzserum für diagnostische Zwecke.**

(57)    Die Erfindung betrifft ein lyophilisiertes Kontroll- oder Referenzplasma bzw. Kontroll- oder Referenzserum für diagnostische Zwecke, welches Lipoproteine, insbesondere Apo-B-hältige Lipoproteine enthält, einen Wassergehalt zwischen 1 und 10 %, vorzugsweise zwischen 1,5 und 5 %, aufweist und zu einer klaren Lösung mit einer Trübung von maximal 70 LSU rekonstituierbar ist. Weiters betrifft die Erfindung ein Verfahren zur Herstellung eines lyophilisierten Kontroll- oder Referenzplasmas bzw. Kontroll- oder Referenzserums sowie ein Verfahren zur Herstellung eines Kontroll- oder Referenzplasmas bzw. Kontroll- oder Referenzserums.

EP 0 617 289 A2

Die Erfindung betrifft ein lyophilisiertes Kontroll- oder Referenzplasma bzw. Kontroll- oder Referenzserum für diagnostische Zwecke, welches Lipoproteine, insbesondere Apo-B-hältige Lipoproteine enthält, sowie ein Verfahren zu dessen Herstellung.

Die Lyophilisierung von wässerigen Lösungen ist eine übliche Methode zur Herstellung von lagerstabilen Präparaten. Dabei wird Wasser aus der gefrorenen Lösung durch Sublimieren im Vakuum entfernt. Der Wassergehalt von lagerstabilen Lyophilisaten beträgt im allgemeinen weniger als 1 % (w/w). Die Haltbarmachung von wässerigen Lösungen durch Lyophilisieren ist jedoch nur dann angebracht, wenn durch den Wasserentzug die physikalischchemischen Eigenschaften der Inhaltsstoffe nicht wesentlich verändert werden.

Die Lyophilisierung von lipoproteinhältigem Plasma oder Serum stellt ein großes Problem dar, da nach Rekonstitution zumeist trübe Lösungen erhalten werden. Lipoproteine können durch die Lyophilisierung denaturiert werden, so daß ihre Bestimmung mit elektrophoretischen oder immunologischen oder optischen Methoden schwierig ist.

Ein gängiges Maß für die Trübung, die eine Lösung aufweist, ist das LSU (ligh scattering unit). Im allgemeinen gilt eine Lösung als trüb, wenn sie bei der Messung im Nephelometer bei 340 nm und einer Schichtdicke von 1 cm ein Meßsignal von mehr als 70 LSU aufweist (Referenz: Wasser).

Aus der Literaturstelle Clin. Chem. (1990), 36, 366-369 geht hervor, daß sich lyophilisiertes Serum aufgrund der Denaturierung von Lipoproteinen nicht als Referenz zur Bestimmung der Lipoproteine eignet. Dies trifft vor allem auf Apo-B-Lipoproteine zu, deren Struktur während der Dehydrierung und Rehydrierung derart verändert wird, daß ihre Löslichkeit herabgesetzt wird, woraus eine Trübung des Serums erfolgt.

Versuche zur Verhinderung der Trübungsbildung durch Zusatz von Stabilisatoren, wie z.B. Saccharose, zum Ausgangsmaterial (Serum) sind in der AT-B-361 135 und in der US-A-4 701 417 beschrieben. Demnach kann die Trübung, ausgedrückt durch die in der Nephelometrie gemessenen LSU, unzulässig hoch sein, so daß diese Präparate nicht als Referenz zur nephelometrischen Bestimmung herangezogen werden können. Eine Lösung gilt als geeignet zur Verwendung bei diagnostischen Methoden, wenn sie eine Trübung von maximal 70 LSU aufweist.

Ein spezielles Verfahren zur Lyophilisierung ist in der US-A-3 928 566 und in der US-A-3 932 943 beschrieben. Durch ein SprayVerfahren werden in Serum oder Plasma kleine Eiskristalle gebildet, wodurch die Erhöhung der Trübung nach Rekonstitution weniger als 20 % ausmacht. Dieses Verfahren ist jedoch sehr aufwendig.

Die Erfindung stellt sich die Aufgabe, die Nachteile der genannten lipoproteinhältigen Lyophilisate zu vermeiden und ein lyophilisiertes Kontroll- oder Referenzplasma bzw. Kontroll- oder Referenzserum für diagnostische Zwecke zur Verfügung zu stellen, welches zu einer klaren Lösung rekonstituierbar ist und durch ein einfaches Verfahren hergestellt werden kann.

Diese Aufgabe wird erfindungsgemäß durch ein lyophilisiertes Kontroll- oder Referenzplasma bzw. Kontroll- oder Referenzserum für diagnostische Zwecke, welches Lipoproteine, insbesondere Apo-B-hältige Lipoproteine enthält, gelöst, welches dadurch gekennzeichnet ist, daß es einen Wassergehalt zwischen 1 und 10 %, vorzugsweise zwischen 1,5 und 5 %, aufweist und zu einer klaren Lösung mit einer Trübung von maximal 70 LSU rekonstituierbar ist.

Ein besonders bevorzugter Bereich des Wassergehaltes des Lyophilisates liegt zwischen 2 und 5 %.

Es hat sich herausgestellt, daß bedingt durch eine bestimmte Restfeuchte im Lyophilisat die Rekonstitution zu einer klaren Lösung möglich ist. Der Wassergehalt im Lyophilisat steht im unmittelbaren Zusammenhang mit der Zunahme der Trübung durch die Lyophilisierung. Je höher der Wassergehalt des hydratisierten Lyophilisates, umso geringer ist die Zunahme der Trübung. Eine Restfeuchte von 1 bis 10 % (w/w) ist vor allem deshalb zweckmäßig, da bei einem höheren Wassergehalt die Lagerstabilität gefährdet sein kann.

Es wurde auch gefunden, daß die Zunahme der Trübung durch die Lyophilisierung und Rekonstitution umso größer ist, wenn bereits das Ausgangsmaterial relativ trüb ist. Dabei ist zu beachten, daß von einem geeigneten Ausgangsmaterial ausgegangen wird, um in der rekonstituierten Lösung nicht mehr als 70 LSU zu messen. Werte über 70 LSU entsprechen einer Trübung, die die Verwendung der Lösung als Referenz bzw. Kontrolle für nephelometrische Bestimmungen praktisch unbrauchbar macht. Besonders gut geeignet sind Werte von bis zu 50 LSU.

Das erfindungsgemäße lyophilisierte Kontroll- oder Referenzplasma bzw. Kontroll- oder Referenzserum kann weiters nicht reduzierende Zucker, wie Saccharose, zur Stabilisierung enthalten. Ein Gehalt an Saccharose von nicht mehr als 25 %, vorzugsweise 12 bis 20 %, ist vorteilhaft.

Das erfindungsgemäße Plasma bzw. Serum ist ausreichend lagerstabil. Dies war nicht vorherzusehen, da eher danach getrachtet wurde, den Wassergehalt in einem Lyophilisat möglich weit herabzusetzen, um dieses lagerstabil zu machen.

Die Erfindung betrifft weiters ein Verfahren zur Herstellung eines lyophilisierten Kontroll- oder Referenzplasmas bzw. Kontroll- oder Referenzserums, welches dadurch gekennzeichnet ist, daß lipoproteinhältigem

2

Plasma bzw. Serum gegebenenfalls ein nicht reduzierender Zucker, vorzugsweise Saccharose, zugesetzt wird und bis zum Erreichen eines Wassergehaltes zwischen 1 und 10 % lyophilisiert wird.

Es hat sich gezeigt, daß eine Trübung von mehr als 70 LSU in der rekonstituierten Lösung dann verhindert werden kann, wenn das Lyophilisat im angefeuchteten Zustand bzw. in feuchter Atmosphäre inkubiert wird. Dabei nimmt das Lyophilisat in reversibler Weise Feuchtigkeit wieder auf, was wiederum eine klare rekonstituierte Lösung zur Folge hat. Auf diese Weise können Lyophilisate, die bei üblicher Rekonstitution (Zugabe von Wasser) eine trübe und daher unbrauchbare Lösung ergeben würden, zu Lösungen rekonstituiert werden, die eine Trübung von weniger als 70 LSU aufweisen und daher in der Diagnostik verwendet werden können.

Ein bevorzugtes Verfahren zur Herstellung eines lyophilisierten Kontroll- oder Referenzplasmas bzw. Kontroll- oder Referenzserums ist daher dadurch gekennzeichnet, daß ein lyophilisiertes lipoproteinhältiges Plasma bzw. Serum in feuchter Atmosphäre inkubiert wird.

Eine weitere Ausführungsform des Verfahrens zur Herstellung eines lyophilisierten Kontroll- oder Referenzplasmas bzw. Kontroll- oder Referenzserums besteht darin, daß zu einem lipoproteinhältigen Plasma oder Serum gegebenenfalls nicht reduzierende Zucker, vorzugsweise Saccharose, zugegeben werden, das Serum oder Plasma daraufhin lyophilisiert wird und das Lyophilisat durch Inkubation in feuchter Atmosphäre auf einen Wassergehalt zwischen 1 und 10 % eingestellt wird.

Es können also auch aus Lyophilisaten mit einem geringen Wassergehalt durch Inkubation in feuchter Atmosphäre Kontroll- oder Referenzplasmen bzw. Kontroll- oder Referenzseren hergestellt werden, die eine Trübung von maximal 70 LSU aufweisen.

Ein Verfahren zur Herstellung eines Kontroll- oder Referenzplasma bzw. Kontroll- oder Referenzserum für diagnostische Zwecke, welches Lipoproteine, insbesondere Apo-B-hältige Lipoproteine enthält, ist dadurch gekennzeichnet, daß ein erfindungsgemäßes Kontroll- oder Referenzplasma bzw. Kontroll- oder Referenzserum zu einer klaren Lösung mit einer Trübung von maximal 70 LSU rekonstituiert wird.

Eine besonderes bevorzugte Ausführungsform eines Verfahrens zur Herstellung eines Kontroll- oder Referenzplasma bzw. Kontroll- oder Referenzserum für diagnostische Zwecke, welches Lipoproteine, insbesondere Apo-B-hältige Lipoproteine enthält, besteht darin, daß ein lyophilisiertes lipoproteinhältiges Plasma bzw. Serum durch Inkubation in feuchter Atmosphäre auf einen Wassergehalt zwischen 1 und 10 % eingestellt wird und anschließend zu einer klaren Lösung mit einer Trübung von maximal 70 LSU rekonstituiert wird.

Die Inkubation in feuchter Atmosphäre erfolgt bei geeigneten Temperaturen im Bereich von 4 bis 50°C, vorzugsweise zwischen 30 und 40°C, insbesondere bei etwa 37°C, so lange, bis die Trübung der rekonstituierten Lösung einem Wert von maximal 70 LSU entspricht. Die Dauer der Inkubation ist temperaturabhängig und kann 1 bis 50 Tage, vorzugsweise 7 bis 28 Tage, betragen. Ein Zusatz von nicht reduzierenden Zuckern, insbesondere Saccharose, kann vor der Lyophilisierung erfolgen.

Die Erfindung wird durch die nachfolgenden Beispiele näher erläutert:

Beispiel 1:

Herstellung von Plasma-Lyophilisat mit unterschiedlichem Wassergehalt

Rethrombinisierte frische Plasmaproben wurden mit 15 % Saccharose versetzt und auf ihre Trübung untersucht. Danach wurden Proben mit 20 LSU bis zu einer Restfeuchte von 0,5; 1,1; 3,8 und 8,5 % (w/w) Wasser lyophilisiert. Zum Vergleich wurden Plasmaproben mit 48 LSU bis zu einer Restfeuchte von 1,1 und 2,8 % (w/w) lyophilisiert. Der Wassergehalt wurde nach der Methode von Karl Fischer gemessen.

Die Lyophilisate wurden mit Wasser rekonstituiert und bis zum Erreichen des Ausgangsvolumens verdünnt. Die Trübung der rekonstituierten Lösungen wurde mit einem IMMUNO Video-Nephelometer (Fa. IMMUNO AG) gemessen.

Tabelle 1 zeigt, daß die Trübung der Lösung des Lyophilisates mit 0,5 % (w/w) Wassergehalt unzulässig hoch ist. Ebenso ist ersichtlich, daß Plasma mit einer höheren Eigentrübung erst bei einem höheren Wassergehalt des Lyophilisates eine noch zulässige Trübung in der rekonstituierten Lösung mit sich bringt. Es besteht ein linearer Zusammenhang zwischen dem Logarithmus des Wassergehaltes und dem Logarithmus des Verhältnisses der Trübung in der rekonstiuierten Lösung zu der des Ausgangsplasmas (r = -0,83).

Tabelle 1

Einfluß des Wassergehaltes im Plasma-Lyophilisat auf die
Trübung in der rekonstituierten Lösung

| Wassergehalt im Lyophilisat rekonstituierter (%, w/w) (LSU) | Trübung im Ausgangsplasma (LSU) | Trübung in Lösung |
|---|---|---|
| 0,5 | 20 | 100 |
| 1,1 | 20 | 70 |
| 3,8 | 20 | 26 |
| 8,5 | 20 | 19 |
| 1,1 | 48 | 100 |
| 2,8 | 48 | 69 |

Beispiel 2:

Prüfung der Lagerstabilität eines Kontrollserums in Abhängigkeit von der Restfeuchtigkeit

Lp(a)-hältige Kontrollseren, hergestellt nach dem Verfahren der AT-B-361 135, mit einem Gehalt an Saccharose (15 %) mit unterschiedlichem Wassergehalt wurden bei 37°C 14, 21 und 28 Tage inkubiert. Erfahrungsgemäß entsprechen 28 Tage bei 37°C einer Lagerstabilität von 2 Jahren bei +4°C. Zur Beurteilung der Stabilität wurde Lp(a) (Lipoprotein a) quantitativ bestimmt und der Quotient $Q_{Lp}(a)$ berechnet.

$$Q_{Lp(a)} = \frac{Lp(a)\text{-Gehalt n Tage } 37°C}{Lp(a)\text{-Gehalt 0 Tage } 37°C}$$

Tabelle 2

| Lagerstabilität eines Kontrollserums mit unterschiedlichem Wassergehalt | | | | |
|---|---|---|---|---|
| Wassergehalt (%) | $Q_{Lp(a)}$ nach einer Inkubationsdauer von | | | |
| | 0 d | 14 d | 21 d | 28 d |
| 3,0 | 1,00 | 1,00 | 1,00 | 1,04 |
| 1,5 | 1,00 | 0,94 | 1,06 | 1,00 |
| 1,0 | 1,00 | 1,07 | 1,05 | 1,04 |
| 0,5 | 1,00 | 0,94 | 0,91 | 0,94 |

Für den untersuchten Zeitraum ist die Lagerstabilität des Lp(a)-hältigen Serums unabhängig von der Restfeuchte.

Beispiel 3:

Herstellung von Serum-Lyophilisat mit unterschiedlichem Saccharosegehalt

Lp(a)-hältiges Kontrollserum, hergestellt nach dem Verfahren der AT-B-361 135, wurde mit verschiedenen Anteilen Saccharose (5 %, 10 %, 15 %) versetzt, eingefroren und so lyophilisiert, daß im Endprodukt der Wassergehalt variierte.

## Tabelle 3

### Einfluß von Saccharosegehalt im Serum-Lyophilisat
### auf die Trübung der rekonstituierten Lösung

| | Trübung (LSU) in der rekonstituierten Lösung des Serum-Lyophilisates mit einem Saccharosegehalt von | | |
| --- | --- | --- | --- |
| Wassergehalt (%) | 5 % | 10 % | 15 % |
| | | Saccharose | |
| 8,0 - 8,4 | 280 LSU | 57 LSU | n.b.* |
| 2,9 - 4,2 | 195 LSU | 95 LSU | 41 LSU |
| 1,0 - 1,2 | 210 LSU | 194 LSU | 73 LSU |

\*) ... nicht bestimmt

Beispiel 4:

Inkubation von Serum-Lyophilisat zur Herabsetzung der Trübung in der rekonstituierten Lösung

2 Chargen von Lp(a)-hältigem Kontrollserum, hergestellt nach dem Verfahren der AT-B-361 135, mit einem Saccharosegehalt von 15 % wurden lyophilisiert und anschließend in feuchter Atmosphäre bei 37°C inkubiert. Die Zunahme des Wassergehaltes im Lyophilisat sowie die Abnahme der Trübung in der rekonstituierten Lösung wurden nach 1, 2, 3 und 4 Wochen bestimmt.

Tabelle 4

| Einfluß der Inkubationsdauer auf den Wassergehalt im Serum-Lyophilisat und auf die Trübung der rekonstituierten Lösung | | | | |
| --- | --- | --- | --- | --- |
| | Charge 1 | | Charge 2 | |
| Inkubationsdauer (d) | Trübung der rek. Lösung (LSU) | Wassergehalt (%) | Trübung der rek. Lösung (LSU) | Wassergehalt (%) |
| 0 | 88 | 1,2 | 270 | 1,7 |
| 7 | 49 | 1,3 | 228 | 2,4 |
| 14 | 38 | 1,4 | 181 | 3,2 |
| 28 | 14 | 2,5 | 40 | 3,6 |

## Patentansprüche

1. Lyophilisiertes Kontroll- oder Referenzplasma bzw. Kontroll- oder Referenzserum für diagnostische Zwecke, welches Lipoproteine, insbesondere Apo-B-hältige Lipoproteine enthält, dadurch gekennzeichnet, daß es einen Wassergehalt zwischen 1 und 10 %, vorzugsweise zwischen 1,5 und 5 %, aufweist und zu einer klaren Lösung mit einer Trübung von maximal 70 LSU rekonstituierbar ist.

2. Lyophilisiertes Kontroll- oder Referenzplasma bzw. Kontroll- oder Referenzserum nach Anspruch 1, dadurch gekennzeichnet, daß es nicht reduzierende Zucker, insbesondere 12 bis 20 % Saccharose, enthält.

3. Verfahren zur Herstellung eines lyophilisierten Kontroll- oder Referenzplasma bzw. Kontroll- oder Referenzserum nach Anspruch 1, dadurch gekennzeichnet, daß lipoproteinhältigem Plasma bzw. Serum gegebenenfalls ein nicht reduzierender Zucker, vorzugsweise Saccharose, zugesetzt wird und bis zum Erreichen eines Wassergehaltes zwischen 1 und 10 % lyophilisiert wird.

4. Verfahren zur Herstellung eines lyophilisierten Kontroll- oder Referenzplasma bzw. Kontroll- oder Referenzserum nach Anspruch 1, dadurch gekennzeichnet, daß ein lyophilisiertes lipoproteinhältiges Plasma bzw. Serum in feuchter Atmosphäre inkubiert wird.

5. Verfahren zur Herstellung eines lyophilisierten Kontroll- oder Referenzplasma bzw. Kontroll- oder Referenzserum nach Anspruch 1, dadurch gekennzeichnet, daß zu einem lipoproteinhältigen Plasma oder Serum gegebenenfalls nicht reduzierende Zucker, vorzugsweise Saccharose, zugegeben wird, lyophilisiert wird und das Lyophilisat durch Inkubation in feuchter Atmosphäre auf einen Wassergehalt zwischen 1 und 10 % eingestellt wird.

6. Verfahren nach einem der Ansprüche 4 oder 5, dadurch gekennzeichnet, daß die Inkubation in feuchter Atmosphäre bei einer Temperatur zwischen 4 und 50°C, vorzugsweise zwischen 30 und 40°C, und während einer Zeitdauer zwischen 1 und 50 Tagen, vorzugsweise 7 bis 28 Tagen, erfolgt.

7. Verfahren zur Herstellung eines Kontroll- oder Referenzplasma bzw. Kontroll- oder Referenzserum für diagnostische Zwecke, welches Lipoproteine, insbesondere Apo-B-hältige Lipoproteine enthält, dadurch gekennzeichnet, daß ein lyophilisiertes Kontroll- oder Referenzplasma bzw. Kontroll- oder Referenzserum nach einem der Ansprüche 1 oder 2 zu einer klaren Lösung mit einer Trübung von maximal 70 LSU rekonstituiert wird.

8. Verfahren zur Herstellung eines Kontroll- oder Referenzplasma bzw. Kontroll- oder Referenzserum für diagnostische Zwecke, welches Lipoproteine, insbesondere Apo-B-hältige Lipoproteine enthält, dadurch gekennzeichnet, daß ein lyophilisiertes lipoproteinhältiges Plasma bzw. Serum durch Inkubation in feuchter Atmosphäre auf einen Wassergehalt zwischen 1 und 10 % eingestellt wird und anschließend zu einer klaren Lösung mit einer Trübung von maximal 70 LSU rekonstituiert wird.

9. Verfahren nach Anspruch 7, dadurch gekennzeichnet, daß die Inkubation in feuchter Atmosphäre bei einer Temperatur zwischen 4 und 50°C, vorzugsweise zwischen 30 und 40°C, und während einer Zeitdauer zwischen 1 und 50 Tagen, vorzugsweise 7 bis 28 Tagen, erfolgt.